# EUROPEAN PATENT APPLICATION

(11) **EP 3 939 689 A1**
(43) Date of publication of application: **19.01.2022**
(21) Application number: 20185952.7
(22) Date of filing: 15.07.2020
(51) Int. Cl.: B01D 53/86, C10L 3/10

(54) **A PROCESS FOR CATALYTIC REDUCTION OF OXYGEN TO A SUB-PPMV LEVEL IN A GAS**

(71) Applicant: Haldor Topsøe A/S, 2800 Kgs. Lyngby (DK)
(72) Inventor: MADSEN, Anders Theilgaard, 2300 Copenhagen (DK); SØRENSEN, Per Aggerholm, 2800 Kgs. Lyngby (DK); JAKOBSSON, Niklas Bengt, 26876 Kågeröd (SE)
(74) Representative: Haldor Topsøe A/S

(57) **Abstract**

In a process for catalytic reduction of oxygen to sub-ppmv level in an oxygen-containing gas, the oxygen reduction is conducted in two consecutive catalytic steps, first with a main reductant and then with H₂ as a secondary reductant. The main reductant is preferably methanol.

## Description

The present invention relates to a process for catalytic reduction of oxygen to a sub-ppmv level in a gas.

In a range of technical gases and gas-upgrading applications, it is important or even necessary to remove oxygen down to very low levels. In many chemical and catalytic processes, oxygen is either a poison, or it may lead to degradation of process equipment, catalysts, reactants or products. Biogases, landfill gases and digester gases all contain oxygen impurities from air ingress, which can amount to several vol-% of oxygen being present in the gas, and which can never be tolerated for upgraded gas qualities such as renewable or substitute natural gas. In addition, natural gas, reduced gases, pyrolysis gases, gasification gases or reforming gases often cannot tolerate oxygen for their downstream processing. These gases normally also contain sulfur compounds, such as hydrogen sulfide, sulfur dioxide, carbonyl sulfide etc. in the ppmv range.

Industrial gases, such as acid gases and CO₂-containing off-gases, cannot be valorized or separated if there is too much oxygen is present. It is therefore necessary to minimize the level of oxygen found, depending on the intended further processing and valorization of these gases. The industrial solutions presently applied are sensitive to the sulfur compounds and often poisoned thereby, thus hampering their applicability.

The present invention solves the problem of sulfur-tolerant removal of oxygen in industrial gases down to ppmv and sub-ppmv levels of O₂ at low or no stoichiometric excess reductant without combusting methane contained in the gas.

The standard solution in the field is to use certain solid absorbers (scavengers) which can remove oxygen down to a level below 0.01 vol% in air. However, such absorbers must be changed once their capacities are spent. Such solid scavengers are typically used in small scale or food grade technical applications.

Various scavengers and reductants, such as dissolved sulfites or ascorbates, can be added in order to reduce oxygen dissolved in water. This is often the case in, e.g., boiler feed- water or the like. Adsorption of components on high-surface area activated carbon or other high-surface area materials is another well-known method for removal of oxygen but, typically, activated carbon is sensitive to oxygen and gets degraded over time, and the high-surface area material will also adsorb many components of higher molecular weight and small polar components, like H₂O, in preference of O₂.

For high-temperature (>600°C) purposes, oxygen-conducting electrochemical cells can be used to remove O₂ down to sub-ppmv levels with an electrical current at a significant over-potential. This is, however, only possible with gases which do not contain combustible components, and compounds containing sulfur cannot be tolerated.

Cryogenic distillations are extensively used to separate air into pure N₂/O₂ technical gas, but this technique is energy intensive and generally undesired if only minor O₂ amounts are to be removed from a gas and the O₂ itself is considered useless.

A few catalytic process solutions for oxygen removal by reduction have been developed. However, these solutions all consider using reductants already present in the gas to remove the oxygen as CO₂ and H₂O.

WO 2008/107709 describes a process for reducing free oxygen in a gaseous hydrocarbon stream, where the gaseous hydrocarbon stream is passed over a material comprising a metal selected from Ni, Co, Cu, Fe, Mn or Ag in a reduced state, so that oxygen present in said stream reacts with the metal. The metal in the reduced state is formed by (i) withdrawing a portion of the hydrocarbon stream, (ii) forming a gas mixture containing hydrogen from the hydrocarbon portion, and (iii) passing the gas mixture containing hydrogen over the material containing the metal in reducible form.

The use of a copper catalyst to remove small amounts of oxygen from a gas stream containing primarily hydrogen and carbon monoxide, but also small amounts of acetylene and ethylene, is described in US 4,034,062.

US 2014/0012060 describes a process and a catalyst for removing oxygen from hydrocarbon streams. The catalyst comprises from 0.01 to 0.5 wt% of platinum, based on the catalyst, and optionally tin, with the weight ratio of Sn:Pt being from 0 to 10, on zeolite A as support.

WO 2017/009243 describes a process for removing oxygen from a gas mixture comprising a hydrocarbon and oxygen, wherein the oxygen is at least partly converted in the presence of a catalyst to CO₂ and H₂O. The catalyst used is an eggshell catalyst composed of a catalyst support in the form of a shaped body with an outer shell comprising Au and one or more precious metals selected from Pa, Pt, Rh, Ir and Ru, each of which may independently be in metallic form, in the form of an alloy or in the form of salts and not comprising any alkali metals or alkali metal salts.

US 5,446,232 discloses a method for removing oxygen from hydrogen, a hydrocarbon or a halogenated hydrocarbon gas which contains about 0.01-10 mole% oxygen. The oxygen removal is accomplished by contacting the gas with a Hop-calyte catalyst (a mixture of copper and manganese oxides) at a temperature of about 100-300°C.

In US 2010/0229466, a process for reducing free oxygen in a gaseous nitrogen stream is described, which comprises the steps of reforming a hydrocarbon to generate a gas mixture containing hydrogen and carbon oxides, mixing the gas mixture with a nitrogen stream containing free oxygen, and passing the resulting nitrogen gas mixture over a conversion catalyst that converts at least a portion of the free oxygen present in the nitrogen to steam, wherein the hydrocarbon reforming step includes oxidation of a hydrocarbon using an oxygen-containing gas.
In a recent patent application by Applicant's landfill gas group, published as US 2019/0126201, a one-step catalytic process is described, in which oxygen removal is furnished by injecting different reductants into the gas and reacting them with available oxygen over Applicant's ScavengOx^{™} catalyst. These catalytic solutions, however, can only reduce the O₂ levels to low-ppmv or sub-ppmv levels if an excess of reductant, whether added to the gas or already present in the gas, is used.

The above-mentioned ScavengOx^{™}catalyst is preferably based on a monolithic substrate material, or it is a pelletized material. The ScavengOx^{™} catalyst is based on a sulfur-tolerant support material, such as the oxides of alumina, titania, silica or ceria, or high surface area carbon, preferably - but not exclusively - TiO₂ or SiO₂. The active phase and the promotors are based on one or more of the following: Iron, vanadium, tungsten, chromium, copper, manganese, molybdenum, platinum, palladium, rhodium, ruthenium, gold or silver in metallic or metal oxide form.

A preferred version of the ScavengOx^{™} catalyst comprises a metal selected among vanadium, tungsten, chromium, copper, manganese, molybdenum, platinum, palladium, rhodium and ruthenium in metallic or metal oxide form supported on a carrier selected from alumina, titania, silica and ceria.

In the method of the invention, the oxygen reduction is conducted in two consecutive catalytic steps instead of one, i.e. first with a main reductant and then with H₂ as a secondary reductant. The novelty is based on Applicant's recent finding that typical biogases, landfill gases and digester gases will lie inside the thermodynamic limits of carbon formation on the catalyst at temperatures below 500°C, if a carbonaceous reductant is used in excess of the amount necessary for complete O₂ conversion to CO₂ and H₂O. Any operation inside this carbon limit will eventually lead to catalyst deactivation by carbon laydown. However, excess carbonaceous reductant is on the other hand necessary to furnish low-ppmv and sub-ppmv levels of O₂ at acceptable contact times in the catalytic reactor. Deactivation and carbon formation have been observed at higher stoichiometric excesses of added reductant.

So the invention concerns a process for catalytic reduction of oxygen to sub-ppmv level in a gas, wherein the oxygen reduction is conducted in two consecutive catalytic steps, first with a main reductant and then with H₂ as a secondary reductant.

Also, H₂ added to the gas, produced in situ in the gas or already present in the gas is reacted with O₂, furnishing reduction of O₂ at any level and down to sub-ppmv levels.

Preferably, the main reductant in the first catalytic step is methanol. It is further preferred that the last minor amount of oxygen remaining after the first catalytic step is reacted with H₂ to H₂O using ScavengOx^{™} as catalyst at a stoichiometric H₂ excess between 0 and 2.5%.

Thus, the O₂ minimization is conducted in two consecutive catalytic steps: The first step by injecting a sub-stoichiometric amount of a suitable carbon-containing reductant, or usinng one already present in the gas, to remove most of the O₂ in the gas as CO₂ and H₂O, and the second step by injecting below 2.5% and preferably down to 0% of a stoichiometric excess of H₂ as reductant and removing the remaining O₂ from the first step, or using H₂ already in or generated in the first step in the gas, or by reacting a sub-stoichiometric amount of H₂ to achieve a certain O₂ concentration in the gas. By using H₂ as reductant, the stoichiometric excess of reductant can be lowered, and the reductant used cannot lead to carbon formation as no carbon atoms are contained in it.

Furthermore, it has been found that SO₂ present in the gas has not reacted with the reductant and does not lower the reaction rate prohibitively for the catalytic process to occur.

By conducting the O₂ reduction in two consecutive catalytic steps, several advantages can be obtained: Most of the O₂ is reacted to CO₂ and H₂O with a cheap and easy-to-dose reductant (such as methanol) without the risk of carbon formation and deactivation in the first step. The last minor amount of O₂ is reacted with H₂ to H₂O, which is possible with the ScavengOx^{™} catalyst at a stoichiometric H₂ excess between 0 and 2.5%, thus avoiding solid carbon formation and deactivation by the reductant in the second step. Furthermore, economical oxygen reduction by stoichiometric or very low excess reductant furnishes low-ppmv and sub-ppmv levels of O₂.

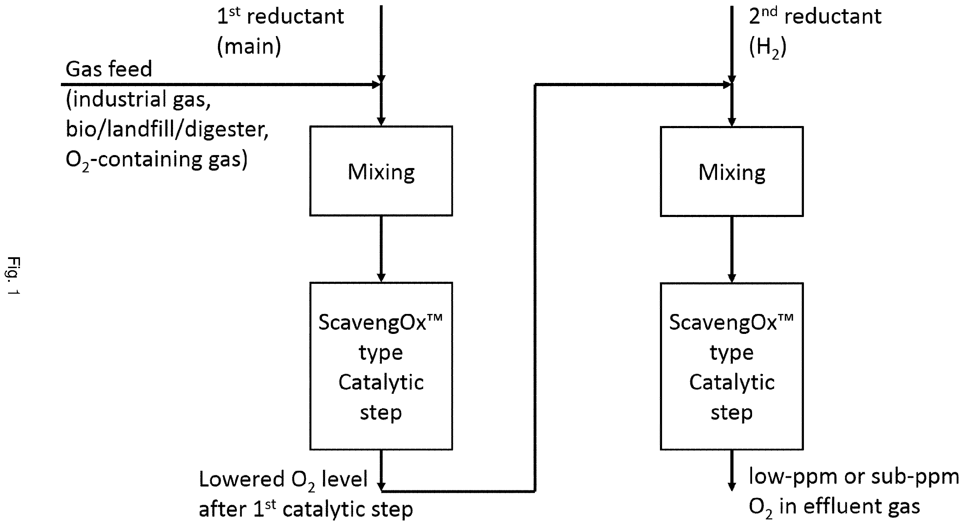

The process according to the invention for catalytic reduction of oxygen to sub-ppmv level in an oxygen-containing gas is illustrated in the figure, which is a flow sheet which shows a two-step O₂ minimization with use of a main first reductant (in the first catalytic step) and a second reductant, H₂ (in the second catalytic step) to obtain a very low O₂ level in the gas.

The method of the invention is illustrated in the example which follows.

### Example

A gas feed of 976 ppmv O₂, 2 vol-% of H₂O and balance N₂ was mixed with 1940-2000 ppmv H₂. The mixing length was more than 100 times the diameter.

The mixed gas feed was heated to reaction temperatures from 250 to 300°C and reacted over a ScavengOx^{™} catalyst. The ratio of normalized flow volume to reactor volume was 2400 Nm³/h/m³. At a stoichiometric H₂ excess from 0.0% to 2.5%, 0.3 ppmv to 0.7 ppmv O₂ at ± 0.2 ppmv O₂ accuracy was always measured in the catalyst effluent.

An addition of 400 ppmv SO₂ to the feed gas did not markedly alter the conversion of the O₂ (only 0.1 ppmv extra O₂ was measured in the effluent from the catalyst).

## Claims

1. A process for catalytic reduction of oxygen to sub-ppmv level in an oxygen-containing gas, wherein the oxygen reduction is conducted in two consecutive catalytic steps, first with a main reductant and then with H₂ as a secondary reductant.

2. Process according to claim 1, wherein the oxygen-containing gas is selected from industrial gases, biogases, landfill gases and digester gases.

3. Process according to claim 1 or 2, wherein the main reductant in the first catalytic step is methanol.

4. Process according to claim 1, wherein the last minor amount of oxygen remaining after the first catalytic step is reacted with H₂ to H₂O using ScavengOx^{™} as catalyst at a stoichiometric H₂ excess between 0 and 5%, preferably between 0 and 2.5%.

5. Process according to claim 4, wherein the ScavengOx^{™} catalyst comprises a metal selected among vanadium, tungsten, chromium, copper, manganese, molybdenum, platinum, palladium, rhodium and ruthenium in metallic or metal oxide form.

6. Process according to claim 4, wherein the ScavengOx^{™} catalyst is supported on a carrier selected from alumina, titania, silica and ceria.

7. Process according to any of the preceding claims, wherein H₂ is in the gas and is partly converted in step 1.

8. Process according to any of the preceding claims, wherein O₂ conversion is furnished partly or completely with H₂ in a gas that has thermodynamic potential for elemental carbon formation.

9. Process according to any of the preceding claims, wherein O₂ reduction is only partly furnished by addition of a sub-stoichiometric amount of H₂.

10. Process according to any of the preceding claims, wherein O₂ reduction is furnished by H₂ formed in situ by reactions of other components in the gas.
